(19) Europäisches Patentamt
European Patent Office
Office européen des brevets

(11) Publication number: 0 209 241 A2

# (12) EUROPEAN PATENT APPLICATION

(21) Application number: 86304428.5

(22) Date of filing: 10.06.86

(51) Int. Cl.⁴: C 07 D 213/08

(30) Priority: 10.06.85 GB 8514611

(43) Date of publication of application: 21.01.87 Bulletin 87/4

(84) Designated Contracting States: GB

(71) Applicant: IEL Limited, ICI House 34, Chowringhee Road, Calcutta 700071 West Bengal (IN)

(72) Inventor: Rajaram, Potaraju, Alchemie Research Centre Private Ltd, CAFI Site, Belapur Road, Thane 400 601, Maharashtra (IN)
Inventor: Joshi, Milind Vishnu, Alchemie Research Centre Private Ltd, CAFI Site, Belapur Road, Thane 400 601, Maharashtra (IN)

(74) Representative: Collier, Jeremy Austin Grey et al, J.A.Kemp & Co. 14, South Square Gray's Inn, London WC1R 5EU (GB)

## (54) Process for the production of pyridines.

(57) Pyridines and pyridine bases are made by the reaction of ethanol with ammonia in the vapour phase at a temperature of 350° to 500°C in the presence of a heteropolyacid catalyst on a gamma-alumina support.

EP 0 209 241 A2

# PROCESS FOR THE PRODUCTION OF PYRIDINES

The present invention relates to the production of pyridines and pyridine bases by the reaction of ethanol with ammonia.

The formation of mono-, di- and triethylamines by the reaction of ethanol with ammonia in the presence of an acidic or a metallic catalyst has been known for many years. One specific method for the production of ethylamines is disclosed at page 454 of Volume 19 of Kirk and Othmer's Encyclopedia of Chemical Technology, third edition. In this known reaction, the yield of ethylamines varies depending upon the type of catalyst employed and the process conditions prevailing. With the background of this knowledge, it is surprising that the same two reactants, viz. ethanol and ammonia, can be used for the production of pyridines provided the catalyst and reaction conditions are correctly chosen.

Pyridines and pyridine bases such as 2-picoline, 3-picoline, 4-picoline and 2-methyl-5-ethyl-pyridine have in the past generally been produced by a cyclo-condensation process in which an aldehyde, such as acetaldehyde, crotonaldehyde or acrolein, is treated with ammonia in the vapour phase at elevated temperature and pressure in the presence of a catalyst. Unfortunately, such cyclo-condensation processes are extremely complicated and because of the high temperatures and pressures essential

for the reaction, the process necessitates the employment of sophisticated, and therefore expensive, equipment. As a result, such processes are extremely uneconomical.

The present invention is directed to a process which overcomes or at least reduces the drawbacks of hitherto known methods for pyridine production and makes it possible to produce pyridines in good yield.

According to the present invention a process for the production of pyridines and pyridine bases comprises reacting ethanol and ammonia in the vapour phase in the presence of a heteropolyacid catalyst on a gamma alumina support at a temperature from 350°C to 500°C, and recovering the pyridines thus produced.

Temperature is a critical parameter in the process of the present invention and by regulating the temperature of the reaction it is possible to optimise the yield of pyridines. For high yields of pyridines, temperatures below 350°C or above 500°C are avoided because below 350°C the reaction gives rise to the formation of undesired amounts of ethylamines while at temperatures above 500°C, the heteropolacid catalyst tends rapidly to become deactivated. The preferred range of temperature for the reaction of the invention is from 400°C to 425°C.

According to a preferred feature of the invention, to produce a still higher yield of pyridines, the ammonia and ethanol are employed in a mole ratio in the range of from 0.5 to 2.5, preferably from 1.8 to 2.2. For

greater efficiency of reaction, the ammonia and ethanol are first preheated, vapourised and mixed together being passed over the catalyst.

To achieve the maximum possible yield of pyridines, the overall space velocity of the reactants is maintained at GHSV (gas hourly space velocity) in the range of 1000 to 2000. Under these conditions, the exit products contain pyridine and alkyl pyridines with greater than 50% molar selectivity as determined by gas chromatography. The alkyl pyridines may then be separated by fractional distillation. However, further increase in the space velocity beyond the range stated above tends to reduce the yield of pyridines because of a reduction in the overall conversion of the starting materials.

The pyridines produced by the process of the present invention can be represented by the structural formula:

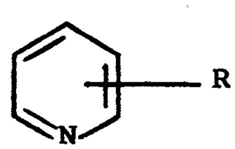

wherein R is a $-CH_3$ group.

The catalyst for the reaction is preferably a heteropolyacid of molybdenum or tungsten wherein the hetero atom is boron, phosphorus or silicon, individually or in any combination. The preferred catalyst is 12-molybdo-phosphoric acid having the chemical structure

$H_3PMo_{12}O_{40}$.

The catalyst must be supported on gamma alumina to ensure a good yield of pyridines and pyridine bases. The catalyst-support combination most preferred is 12-molybdo-phosphoric acid on gamma alumina.

Practical considerations such as convenience and efficiency govern the loading of the catalyst on the support. In practice, the support may be loaded with from 10 to 35 weight per cent. of the heteropolyacid catalyst. More preferably, the loading is generally from 20 to 30 weight per cent. of the catalyst based on the weight of the support.

The process of the invention can be carried out conveniently at ambient temperature as a continuous process. A tubular reactor into which the catalyst is packed as a bed is generally preferred. The reactants, ethanol and ammonia, are preheated, vapourised and mixed, and brought into contact with the preheated catalyst within the reactor. The precise contact or residence time may vary depending on the specific catalyst employed and also in relation to the quantities of the reagents but a minimum contact time of about 1 second, preferably of about 5 seconds, is necessary. A contact time in excess of 20 seconds is seldom necessary and generally the time never exceeds 50 seconds.

The pyridines produced by the reaction may be separated from other reaction products in known manner by

fractional distillation or chromatography.

The invention is described in greater detail in the following Examples:

EXAMPLE I

100 g of gamma alumina granules were placed in a pan noduliser and sprayed with an 8.6 x $10^{-3}$M solution of 12-molybdophosphoric acid (P:Mo:1:12) in acetonitrile till the granules were soaking wet. These granules were dried in air for 12 hours in an oven at 110°C for 2 hours and then activated in a stream of air in a muffle furnace at 500°C for a further 2 hours. The catalyst thus prepared contained 20% by weight of 12-molybdo-phosphoric acid and was identified by X-ray powder pattern (see Chem. Lett. 1981. p.1867).

A tubular catalyst reactor of 25 mm O.D. capable of being heated externally was packed with 5 ml. of 6 to 12 mesh granular catalyst prepared as described above. The temperature of the catalyst tube was gradually raised to 400°C and was maintained isothermally at this value. Ethanol (0.05 ml/min) and ammonia gas (40 ml/min) were vaporised and preheated separately and fed into the catalytic reactor so that the temperature of the reactor remained constant. The products coming from the reactor were fed into an on-line gas chromatography having a Porapak Q column for analysis. It was observed that 94% (molar) alcohol fed into the reactor were converted with a selectvity of greater than 55% (molar) into 2-picoline and 4-picoline.

EXAMPLE II

100 g of gamma alumina granules were placed in a pan noduliser and sprayed with a 1.0 x $10^{-2}$ M solution of 12-tungstosilica acid (Si:W:1:12) in acetonitrile till the granules were soaking wet. These granules were dried in air for 12 hours in an oven at 110°C for 2 hours and then activated in a stream of air in a muffle furnace at 500°C for a further 2 hours. The catalyst thus prepared contained 20% by weight of 12-tungstosilicic acid and was identified by X-ray powder pattern (see Chem. Lett. 1981. p.1867).

A tubular catalytic reactor was packed with 5 ml of 6 to 12 mesh granular catalyst prepared as described above. The temperature of the catalyst tube was gradually raised to 425°C and was maintained isothermally at this value. Ethanol (0.05 ml/min) and ammonia gas (40 ml/min) were vaporised and preheated separately and fed into the catalytic reactor so that the temperature of the reactor remained constant. The products coming from the reactor were fed into an on-line gas chromatography having a Porapak Q column for analysis. It was observed that 90% (molar) alcohol fed into the reactor waas converted with a selectivity of greater than 45% (molar) into 2-picoline and 4-picoline.

## CLAIMS

1. A process for the production of pyridines and pyridine bases which comprises reacting ethanol and ammonia in the vapour phase in the presence of a heteropolyacid catalyst on a gamma-alumina support at a temperature from 350°C to 500°C and recovering the pyridines thus produced.

2. A process as claimed in Claim 1 wherein said reaction is effected at a temperature of from 400°C to 425°C.

3. A process as claimed in Claim 1 wherein the ammonia and ethanol are employed in a mole ratio from 0.5 to 2.5.

4. A process as claimed in Claim 3 wherein the ammonia and ethanol are employed in a mole ratio of from 1.8 to 2.2.

5. A process as claimed in any of Claims 1 to 4 wherein the ammonia and ethanol are preheated, vaporised and mixed together before being passed over said catalyst.

6. A process as claimed in any of Claims 1 to 5 wherein the overall space velocity of the reactants is maintained at a gas hourly space velocity in the range of 1000 to 2000.

7. A process as claimed in any of Claims 1 to 6 wherein said catalyst is a heteropolyacid of molybdenum or tungsten wherein the hetero atom is boron, phosphorus or silicon, individually or in any combination.

8. A process as claimed in Claim 7 wherein said catalyst is 12-molybdo-phosphoric acid having the formula $H_3PMo_{12}O_{40}$.

9. A process as claimed in any of Claims 1 to 8 wherein the contact time of the reagents with the catalyst is from 1 to 50 seconds.

10. A process as claimed in Claim 9 wherein the contact time varies from 5 to 20 seconds.